# EUROPEAN PATENT SPECIFICATION

(11) **EP 3 411 004 B1**
(45) Date of publication and mention of the grant of the patent: **10.03.2021**
(21) Application number: 17702164.9
(22) Date of filing: 23.01.2017
(51) Int. Cl.: A61J 1/10, A61K 9/08, A61L 2/07, A61K 31/167

(54) **PROCESS OF MANUFACTURING A STABLE, READY TO USE INFUSION BAG FOR AN OXIDATION SENSITIVE FORMULATION**
VERFAHREN ZUR HERSTELLUNG EINES STABILEN, GEBRAUCHSFERTIGEN INFUSIONSBEUTELS FÜR EINE OXIDATIONSEMPFINDLICHE FORMULIERUNG
PROCÉDÉ DE FABRICATION D'UNE POCHE POUR PERFUSION INTRAVEINEUSE STABLE ET PRÊTE À L'EMPLOI POUR UNE FORMULATION SENSIBLE À L'OXYDATION

(30) Priority: 05.02.2016 US 201662291589 P
(43) Date of publication of application: 12.12.2018
(73) Proprietor: InnoPharma, Inc., New York, NY 10017 (US)
(72) Inventor: HINGORANI, Tushar, Piscataway, New Jersey 08854 (US); KUNADHARAJU, Sasank Chaitanya, Edison, New Jersey 08817 (US); MALKAN, Tushar, INDIAN CREEK IL 60061-3271 (US); PEJAVER, Satish, Bridgewater, New Jersey 08807 (US)
(74) Representative: Pfizer
(86) International application number: PCT/IB2017/050341
(87) International publication number: WO 2017/134540

(56) References cited:
- EP-A1- 1 889 607
- EP-A1- 2 377 516
- WO-A1-2014/083071
- WO-A1-2014/168895
- WO-A1-2016/013049
- US-A- 6 028 222

## Description

### FIELD OF THE INVENTION

The present invention relates to the process of manufacturing a stable ready to use formulation of an oxidation sensitive drug formulation without deoxygenation of the vehicle or without use of a nitrogen blanket during manufacture of the formulation. In the formulations prepared according to the processes disclosed herein, the amount of the highest impurity generated even at ambient conditions is significantly lower than acetaminophen solution autoclaved in glass bottles and bubbled with nitrogen to give dissolved oxygen levels less than 2 ppm.

### BACKGROUND OF THE INVENTION

The drug product disclosed herein relates to a stable, ready to use parenteral drug product prepared by moist heat sterilization of a drug solution comprising an active pharmaceutical ingredient (API) in a flexible infusion bag, wherein the API in the drug product is susceptible to oxidation by ambient oxygen, light, or moisture, where the manufacturing of the drug product does not involve the use of deoxygenation of the vehicle or use of nitrogen blanket during manufacture of the formulation.

Oxidation of inorganic and organic compounds occurs by a loss of electrons and a loss of a molecule of hydrogen. Alcohols, aldehydes, ketones, alkynes, alkenes, sulfides, thiols, carboxylic acids, benzoins, phenols, quinones, alkylbenzenes, imines, epoxides, catechols, ethers, and organometallics are examples of oxidizable functional groups. These functional groups are found in pharmaceutical compounds such as acetaminophen, acetylcysteine, amikacin sulfate, dopamine hydrochloride, promethazine hydrochloride, linezolid, and in classes of compounds such as amino acids.

Acetaminophen, also referred to as paracetamol or N-(4-hydroxyphenyl)acetamide, is a non-steroidal analgesic and an antipyretic widely used via various routes and is represented as shown in the formula below.

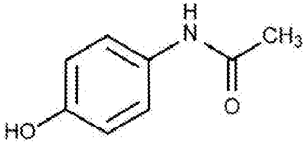

Acetaminophen administered by an intravenous route has a faster on-set and results in more predictable pharmacokinetics than oral or rectal acetaminophen formulations. In a study where six adult volunteers were given intravenous, oral, and rectal acetaminophen, the mean intravenous Cₘₐₓ observed was nearly two and four fold higher compared to administration by an oral route and a rectal route respectively. The intravenous treatment group showed consistently better on-set and higher peak plasma and cerebrospinal fluid (CSF) maximum concentration values with less variability than after either oral or rectal administration.

An advantage of intravenous acetaminophen is that the intravenous acetaminophen may be administered before or during surgery, permitting the initiation of an effective analgesic therapy in an early phase of a postoperative period. Intravenous acetaminophen appears to avoid first pass hepatic exposure and metabolism via portal circulation, which may reduce the potential for hepatic injury. With therapeutic dosing, for example, with up to 4,000 mg daily, intravenous acetaminophen is rarely associated with hepatotoxicity and has been shown to be safe for use in some patients with underlying liver conditions. Nonetheless, according to its prescribing information, intravenous acetaminophen is contraindicated in patients with severe hepatic impairment or severe active liver disease. Advantages of the acetaminophen injection are well known in the art.

Acetaminophen is a *p*-aminophenol derivative, which is synthesized by acetylation of *p*-aminophenol with acetic anhydride. Acetaminophen may be hydrolyzed to *p*-aminophenol at an elevated temperature and in the presence of an acidic medium or a basic medium. *p*-aminophenol is a major impurity in acetaminophen preparations that may be formed during the storage or synthesis of acetaminophen. It was reported that *p*-aminophenol may cause nephrotoxicity and teratogenicity; therefore, the amount of *p*-aminophenol should be strictly controlled. The United States and British pharmacopeias limit the amount of *p*-aminophenol in an acetaminophen substance at 0.005% w/w.

The degradation of acetaminophen in an aqueous solution is both an acid catalyzed reaction and a base catalyzed reaction. It is first order with respect to the concentration of acetaminophen and first order with respect to hydrogen and hydroxyl ion concentration. The half-life for acetaminophen in a buffered solution at pH 5 and pH 6 was calculated to be 19.8 years and 21.8 years respectively. At pH 2, the half-life is 0.73 years, and at pH 9, the half-life is 2.28 years, with intermediate values at intermediate pHs. While formulating acetaminophen in pharmaceuticals, it is desirable to keep the pH of the medium between about 5 to about 6 to maximize the shelf life for the product.

Dietlin and Fredj, U.S. Patent No. 6,028,222, describe an acetaminophen dispersion prepared by using free radical scavengers and/or a radical antagonist, and by bubbling an inert gas through the aqueous solvent to remove oxygen from the medium to maintain the stability of the formulation and prevent the oxidation of the acetaminophen in the formulation.

U.S. Patent Application Publication No. 20040054012 discloses a method for obtaining aqueous formulations of active principles of a phenolic nature susceptible to oxidation. These formulations are prepared by bubbling with at least one inert gas and/or placing under vacuum. These formulations are kept under an inert gas atmosphere or are filled, under inert gas, into bottles previously cleared of air by insufflation with inert gas and by addition of an antioxidant.

U.S. Patent Application Publication No. 20140303254 discloses processes for minimizing formation of a highest degradation product during moist heat sterilization of a drug solution of an oxidation susceptible API, wherein the API is mixed with deoxygenated water to prepare a non-sterile drug solution. The non-sterile drug solution is filled into a moist heat sterilizable flexible infusion bag, and the infusion bag with the non-sterile drug solution is terminally moist heat sterilized at a preset air overpressure between about 0.2 bar to about 1.2 bar to obtain a parenteral drug product.

Terminal sterilization is the method of choice for sterilization of thermally stable APIs. To achieve sterility of a non-sterile drug solution, the non-sterile drug solution must be sterilized in an autoclave to obtain a minimum 6 log reduction of microbial bioburden in the non-sterile drug solution. Each log reduction (10⁻¹) represents a 90% reduction in the microbial bioburden. Therefore, a process shown to achieve a "6 log reduction" (10⁻⁶) will reduce the microbial bioburden from a million organisms (10⁶) to very close to zero, theoretically. It is common to employ an overkill cycle to provide maximum assurance of sterility for critical products such as parenteral solutions, implantable devices, etc. The 6 log reduction is achieved by sterilizing the non-sterile drug solution for at least 15 minutes at 121° C. (250° F.) at 100 kPa (15 psig), or for at least 3 minutes at 134° C. (273° F.) at 100 kPa (15 psig). Additional sterilization time is typically required where the non-sterile drug solution and instruments are packed within an overwrap, as they may take longer to reach the required sterilization temperature.

The acetaminophen API is susceptible to oxidation. An autoclave cycle of acetaminophen in the presence of oxygen leads to the formation of dimer and polymeric impurities, where the acetaminophen drug solution is between a pH of about 5 to about 6. To minimize degradation of the API and the generation of impurities during terminal sterilization, different approaches have been taken. In one approach, the water used for compounding the acetaminophen is deoxygenated and the acetaminophen drug solution is thereafter terminally sterilized in non-oxygen permeable glass bottles in the presence of antioxidants. Conventionally, fluids for parenteral administration to the blood stream of patients have been packaged in glass containers. However, manufacturing and transport of glass containers is challenging. Industrial efforts have been made to find alternative polymeric materials which are less resource consuming, cheaper, and more convenient to handle than glass.

Generally, during processing of an oxidation susceptible API to a parenteral dosage form, the API undergoes degradation by heat to which the API is exposed during terminal moist heat sterilization. ICH guidelines for parenteral formulations require the unknown impurity to be identified. The maximum allowable amount of the impurity depends on the concentration of the daily dose. For example, if the daily dose is 1-10 mg, the identification limit is 0.5% by weight of the API; if the daily dose is greater than 2 gm per day, the identification limit is 0.1 % by weight of the API.

In a moist heat sterilization cycle, air overpressure is typically set at about 1.3 bar to about 1.4 bar to prevent the contents including the drug solution in the flexible infusion bag from expanding and bursting the flexible infusion bag during sterilization. Also, in a moist heat sterilization cycle of an oxidation susceptible API at an air overpressure set at about 1.4 bar, the degradation of the oxidation susceptible API may exceed 0.1% by weight of the labeled amount of the oxidation susceptible API in the drug product in the parenteral dosage form.

Conventional formulations and processes use several excipients and packaging to stabilize the formulation, but fail to address degradation of the API during terminal sterilization. Hence, there is a long felt but unresolved need for reducing the degradation of oxidation susceptible formulations during terminal moist heat sterilization. Furthermore, there is a need for a stable, oxidation susceptible drug solution contained in flexible infusion bags. Furthermore, there is a need for a process for manufacturing a stable, ready to use, oxidation susceptible drug product in a flexible infusion bag that precludes or reduces the oxidation and degradation of the oxidation susceptible API during terminal moist heat sterilization.

From the prior art it is understood that for liquid formulations of acetaminophen, formulation deoxygenation, application of a blanket of inert gas, or purging of nitrogen is required to maintain the stability of the liquid formulation of acetaminophen during and after sterilization. There is a long felt but unmet need for a process of manufacturing a stable, ready to use, oxidation susceptible drug product in a flexible infusion bag without using an inert gas during or after preparation of the formulation, or introducing an inert gas in the formulation.

### DETAILED DESCRIPTION OF THE INVENTION

This summary is provided to introduce a selection of concepts in a simplified form that are further disclosed in the detailed description of the invention. This summary is not intended to identify key or essential inventive concepts of the claimed subject matter, nor is it intended for determining the scope of the claimed subject matter.

The present invention relates to processes of manufacturing a stable, ready to use formulation of an oxidation sensitive drug formulation without deoxygenation of the vehicle or use of nitrogen blanket during formulation or with storing the formulation in ambient conditions in the polymer bag and autoclaving.

In one embodiment of the present invention is provided a process for the manufacture of a pharmaceutically acceptable product comprising an oxidation susceptible API which can be moist heat sterilized in a moist heat sterilizable container, said process comprising: sterilizing a drug solution comprising an oxidation-susceptible API dissolved in water, said drug solution being contained in a moist heat sterilizable container, said moist heat sterilization being performed at an air overpressure between about 0.2 bar to about 1.2 bar, to obtain a parenteral drug product with a degradation of less than 0.1% by weight of a labeled amount of said oxidation susceptible API; wherein said moist heat sterilizable container is a flexible infusion bag made of a plastic material; wherein the water is not deoxygenated and a nitrogen blanket is not used during formulation, or the formulation is stored in ambient conditions in the polymer bag and autoclaved; and wherein said plastic material is a cycloolefinic polymer, a polypropylene polymer, a polyvinyl chloride polymer, or any combination thereof.

In another embodiment of the present invention is provided a parenteral drug product comprising a solution of an oxidation susceptible API and one or more excipients, wherein said solution has a highest degradation product at a level less than 0.1% by weight of a labeled amount of said oxidation susceptible API, wherein said solution is sterilized by moist heat sterilization at an air overpressure between about 0.2 bar to about 1.2 bar; and wherein the water is not deoxygenated and a nitrogen blanket is not used during formulation, or the formulation is stored in ambient conditions in the polymer bag and autoclaved.

In another embodiment of the present invention is provided a process for the manufacture of a stable, ready-to-use parenteral drug product comprising an oxidation susceptible API, said process comprising: providing a moist heat sterilizable container made of a flexible material; filling an oxidation susceptible drug solution in said manufactured moist heat sterilizable container; and sterilizing said oxidation susceptible drug solution filled in said manufactured moist heat sterilizable container in an autoclave at an air overpressure between about 0.2 bar to about 1.2 bar, wherein a highest degradation product in said stable, ready to use parenteral drug product is less than 0.1% by weight of a labeled amount of said oxidation susceptible API, and wherein the water is not deoxygenated and a nitrogen blanket is not used during formulation, or the formulation is stored in ambient conditions in the polymer bag and autoclaved.

In another embodiment of the present invention, the flexible infusion bag filled with the non-sterile drug solution, said solution prepared as described hereinabove, is enclosed within one or more overwraps to preclude the ingress of oxygen, moisture, and/or light to the non-sterile drug solution within the flexible infusion bag during terminal moist heat sterilization and post sterilization during storage of the drug product. The flexible infusion bag with or without an overwrap and with the non-sterile drug solution is moist heat sterilized to obtain a 6 log reduction, minimum, of microbial bioburden in the non-sterile drug solution in the flexible infusion bag.

In another embodiment, the flexible infusion bag filled with the non-sterile drug solution prepared as described hereinabove is moist heat terminally sterilized in an autoclave. The sterilized, flexible infusion bag with the oxidation susceptible drug solution is enclosed within an overwrap in a class 10,000 or class 100,000 clean room to preclude ingress of oxygen, moisture, and/or light into the oxidation susceptible drug product during storage of the drug product. In an embodiment, the sterilized, flexible infusion bag with the oxidation susceptible drug product is enclosed within an overwrap under a class 100 or a class 10,000 laminar flow hood in a class 100,000 clean room.

In another embodiment, the flexible infusion bag, or the flexible infusion bag with the overwrap, containing the oxidation susceptible drug solution prepared as described hereinabove is sterilized by a water cascade sterilization method or steam sterilization at a temperature and a cycle time configured to obtain a minimum of 6 log reduction of the microbial bioburden in the oxidation susceptible drug solution, for example, sterilized at a minimum temperature of about 121° C. for a preset time of, for example, between about 10 minutes to about 30 minutes with an air overpressure set at a pressure between about 0.2 bar to about 1.2 bar, for example, about 0.7 bar. The flexible infusion bag may be made of a cycloolefinic polymer, a polypropylene polymer, a polyvinyl chloride polymer, etc. An example of a flexible infusion bag is the Technoflex® infusion bag of Technoflex Société Anonyme a Directoire. In an embodiment, the flexible infusion bag comprises composite layers of one or more of a minimum of two polymeric materials. In another embodiment, the flexible infusion bag comprises one or more than one compartment. In an embodiment, the flexible infusion bag comprises one or more than one port.

The stable, ready to use parenteral drug product disclosed herein generally relates to a stable product of an oxidation susceptible drug solution comprising an oxidation susceptible API in any suitable therapeutically effective amount, where the oxidation susceptible API has one or more oxidizable functional groups comprising an alcohol, an aldehyde, a ketone, an alkyne, an alkene, a sulfide, a thiol, a carboxylic acid, benzoin, phenol, quinone, alkylbenzene, imines, epoxides, catechols, ethers, and organometallics. The stable, ready to use parenteral drug product disclosed herein generally also relates to a stable product of the oxidation susceptible API with one or more oxidizable functional groups in pharmaceutical compounds such as acetaminophen, acetylcysteine, amikacin sulfate, dopamine hydrochloride, promethazine hydrochloride, linezolid, oxytocin, etc. In an embodiment, the stable, ready to use parenteral drug product disclosed herein comprises one oxidation susceptible API along with one or more APIs not susceptible to oxidation.

The oxidation susceptible drug solution comprises one or more excipients. For example, a formulation of the oxidation susceptible drug solution comprises a vehicle. In an embodiment, the vehicle used is a mixture of a minimum of two solvents. In another embodiment, the vehicle comprises one or more of water, alcohols, glycols, dimethylacetamide N-methylpyrollidone, dimethyl sulfoxide, etc. In another embodiment, the excipients comprise, for example, one or more of water, alcohols, glycols, dimethylacetamide, N-methylpyrollidone, dimethyl sulfoxide, etc.

In another embodiment, the formulation of the oxidation susceptible drug solution comprises buffering excipients. In an embodiment, the buffering excipient comprises one or more of an acetate buffer, a citrate buffer, a borate buffer, a phosphate buffer, a maleic buffer, a succinic buffer, a tartaric buffer, a phthalate buffer, a formate buffer, and a tris buffer. In another embodiment, the buffers are present at a concentration of, for example, about 2 millimolar (mM) to about 500 mM. For example, the buffers are present at a concentration of about 80 mM, at about 40 mM, at about 20 mM, at about 10 mM, or at about 5 mM. For example, in an embodiment, the acetaminophen drug solution comprises about 2 mM to about 500 mM of at least one of an acetate buffer, a citrate buffer, a borate buffer, a phosphate buffer, a maleic buffer, a succinic buffer, a tartaric buffer, a phthalate buffer, a formate buffer, a tris buffer, or any combination thereof.

In another embodiment, the oxidation susceptible drug solution comprises tonicity excipients. In an embodiment, a formulation of the oxidation susceptible drug solution comprises, for example, one or more of about 0.1% to about 1.5% w/v of sodium chloride, about 0.1% to about 1.5% w/v of potassium chloride, about 0.1% to about 1.5% w/v of calcium chloride, about 1% to about 20% w/v of sugars such as dextrose, about 0.1% to about 10% w/v of propylene glycol, and about 0.1 to about 10% w/v of glycerol. The tonicity excipients are present in an amount to make the drug product isotonic to blood.

The pH of the oxidation susceptible drug solution is adjusted to a pH of between 1 and 14. For example, in an embodiment, the pH of the oxidation susceptible drug solution has a pH of between 4 and 8. In another embodiment, the pH of the oxidation susceptible drug solution has a pH between 5.40 and 5.60. In an embodiment, the pH of the acetaminophen drug solution is in a range of about 5 to about 6.

In another embodiment, the flexible infusion bag is overwrapped within one or more overwraps prior to sterilization of the oxidation susceptible drug solution in the flexible infusion bag. In an embodiment, the overwrap is a barrier layer configured to reduce or preclude permeation of oxygen to the oxidation susceptible drug solution contained within the flexible infusion bag during or after sterilization. In another embodiment, the overwrap is a barrier layer configured to reduce or preclude permeation of moisture to the oxidation susceptible drug solution contained within the flexible infusion bag during or after sterilization. In another embodiment, the overwrap is a barrier layer, for example, a plastic foil or an aluminum foil configured to reduce or preclude permeation and ingress of light to the oxidation susceptible drug solution contained within the flexible infusion bag during or after sterilization. In another embodiment, the overwrap is a barrier layer, for example, an aluminum overwrap configured to preclude permeation and ingress of oxygen, moisture, and light. In an embodiment, the oxidation susceptible drug solution is filled in the flexible infusion bag overwrapped with a minimum of one overwrap, for example, an aluminum overwrap along with a minimum of one oxygen scavenger such as D-100 FreshPax® of Multisorb Technologies, Inc., Pharmakeep® KH-500 of Mitsubishi Gas Chemical Company, Inc., etc. In another embodiment, the oxygen scavenger is in the form of a powder, canisters, sheets films, and packets. In another embodiment, the oxidation susceptible drug solution is filled in the flexible infusion bag overwrapped with a minimum of one overwrap along with a minimum of one moisture scavenger, for example, the Zoldine® moisture scavenger of the Dow Chemical Company, the Sylosiv® moisture scavenger of W. R. Grace & Co. Conn., etc. In an embodiment, the moisture scavenger is in the form of a powder, canisters, sheets films, and packets. In an embodiment, the overwrap is, for example, a Polialuvel® overwrap with an oxygen permeability of about <0.01 [cm³/(m²*d*bar)] and water vapor permeability of about <0.01 [g/(m²*d)], Wipf® AG of WIPF Management AG Corporation.

In another embodiment, the oxidation susceptible drug solution in the flexible infusion bag is moist heat sterilized with a minimum of one overwrap. In another embodiment, the flexible infusion bag containing the oxidation susceptible drug solution is moist heat sterilized with a minimum of one overwrap or with one or more overwraps, wherein the overwrap comprises one or more oxygen scavengers and/or moisture scavengers configured to provide a barrier to ingress of oxygen, moisture, and/or light to the oxidation susceptible drug solution within the flexible infusion bag.

In the stable, ready to use parenteral drug product disclosed herein, the volume of the oxidation susceptible drug solution filled in the flexible infusion bag is, for example, between about 10 mL and about 5000 mL. For example, the volume of the oxidation susceptible drug solution is between about 50 mL and about 1000 mL. In another embodiment, the volume of the oxidation susceptible drug solution in the flexible infusion bag is between about 80 mL and about 120 mL. In an embodiment, the strength of the acetaminophen in the parenteral acetaminophen drug product is 10 mg/mL.

In another embodiment, the flexible infusion bag filled with the oxidation susceptible drug solution is terminally sterilized by moist heat sterilization at a minimum temperature of about 80° C. In another embodiment, the flexible infusion bag filled with the oxidation susceptible drug solution is terminally sterilized by moist heat at a minimum temperature of about 90° C. In another embodiment, the flexible infusion bag filled with the oxidation susceptible drug solution is terminally sterilized by moist heat at a minimum temperature of about 100° C. In another embodiment, the flexible infusion bag filled with the oxidation susceptible drug solution is terminally sterilized by moist heat at a minimum temperature of about 121° C. for a time period between about 5 minutes and about 20 minutes.

In another embodiment, the oxidation susceptible drug product disclosed herein is a ready to use, parenteral solution of acetaminophen, wherein the highest degradation product in the acetaminophen drug product is less than 0.5% by weight of the labeled amount of acetaminophen in the parenteral acetaminophen drug product. In an embodiment, the highest degradation product in the ready to use parenteral acetaminophen drug product is not more than about 0.1% of any highest impurity, for example, not more than about 0.08% of any highest impurity, not more than about 0.050% of any highest impurity, not more than about 0.035% of any highest impurity, or not more than about 0.010% of any highest impurity of the oxidation susceptible acetaminophen API in the parenteral acetaminophen drug product.

### EXAMPLE 1

The effect of dissolved oxygen on the stability of acetaminophen solutions (prepared as described in Table 1) in glass bottles during autoclave was determined by preparing the solution in water containing different amounts of dissolved oxygen.

| **TABLE 1: COMPOSITION OF ACETAMINOPHEN INJECTION, 10 MG/ML, 100 ML** | |
|---|---|
| **Constituent** | **Quantity** |
| Acetaminophen, USP | 1000 mq |
| Citric Acid, USP | 193 mg |
| Sodium Chloride, USP | 640 mq |
| Sodium Hydroxide Solution, NF | To adjust pH to 5.5 |
| Hydrochloric acid, NF | To adjust pH to 5.5 |
| Water for injection, USP | q.s. 100 mL |

**Method 1:** Water to be used for compounding was deoxygenated by bubbling nitrogen until the dissolved oxygen level was found to be less than 2 parts per million (ppm). About 90 percent of final water required for drug product was taken in the compounding vessel. A constant headspace of nitrogen was maintained in the compounding vessel. Sodium chloride was added and the solution was mixed until it dissolved completely. Citric acid was added and the solution was mixed until it dissolved completely. The pH was adjusted to about 5.5 using sodium hydroxide. Temperature of the solution was increased to about 40 °C by heating. Acetaminophen API was added and the mixing was continued until a clear solution was obtained. The heat was turned off and the solution was allowed to reach room temperature. The final volume was made up using water deoxygenated as described above to less than 2 ppm. About 100 mL of solution was filled in 100 mL media bottles. Dissolved oxygen was checked using a Mettle Toledo portable dissolved oxygen meter. The media bottle was autoclaved in a Tuttnauer® Brinkmann® autoclave at 121 °C for 20 minutes.

**Method 2:** An Acetaminophen solution was prepared as described above but the dissolved oxygen level of the water used for compounding was deoxygenated to between 3 ppm and 5 ppm. About 100 mL of the solution was filled in 100 mL media bottled and autoclaved at 121 °C for 20 minutes.

**Method 3:** An Acetaminophen solution was prepared as described above but the dissolved oxygen level of the water used for compounding was not deoxygenated; the ambient dissolved oxygen level was 9.18 ppm. About 100 mL of the solution was filled in 100 mL media bottle and autoclaved at 121 °C for 20 minutes.

Impurity levels were determined for each solution before and after autoclaving, using HPLC. Results for Methods 1, 2, and 3 are summarized in Table 2 below.

| **TABLE 2: IMPURITY LEVELS** | | | | | | |
|---|---|---|---|---|---|---|
| **MEDIA BOTTLE** | **Method 1** (N₂ headspace; dissolved O₂ 0.15 ppm) | | **Method 2** (N₂ headspace; dissolved O₂ 3.51 ppm) | | **Method 3** (ambient; dissolved O₂ 9.18 ppm) | |
| | Pre-autoclave | Post-autoclave | Pre-autoclave | Post-autoclave | Pre-autoclave | Post-autoclave |
| **Assay (%)** | 101.9 | 103.5 | 102.0 | 102.9 | 102.3 | 103.7 |
| **Highest Unknown** I**mpurity (%)** | 0.008 | 0.079 | 0.009 | 0.098 | 0.009 | 0.113 |
| **p-aminophenol (%)** | ND | ND | ND | ND | ND | ND |
| **Total Impurities (%)** | 0.008 | 0.133 | 0.009 | 0.163 | 0.009 | 0.186 |
| ND: not detected | | | | | | |

In glass containers, highest unknown impurity increased as dissolved oxygen level increased. The amount of highest impurity at ambient condition is about 1.5 times that of about nitrogen bubbled solution.

### EXAMPLE 2

The effect of dissolved oxygen on stability of acetaminophen solution in polypropylene bags during autoclave was determined by preparing the solution in water containing different amounts of dissolved oxygen.

**Method A:** Acetaminophen solution of the above composition was prepared with water containing dissolved oxygen level less than 2 ppm. About 100 mL of the solution was filled in polypropylene bags and stoppered with a polypropylene end connector. The solution was autoclaved at 121 °C for 20 minutes in a steam sterilizer.

**Method B:** Acetaminophen solution of the above composition was prepared with water with no nitrogen bubbling. About 100 mL of the solution was filled in polypropylene bags and stoppered with a polypropylene end connector. The solution was autoclaved at 121 °C for 20 minutes in a steam sterilizer.

Results are summarized below.

| **TABLE 3: IMPURITY LEVELS** | | | | |
|---|---|---|---|---|
| **IV Bags** | **Method B** (Ambient; dissolved O₂ ∼8-10 ppm) | | **Method A** (N₂ headspace; dissolved O₂ < 2 ppm) | |
| | **Pre-Autoclave** | **Post-Autoclave** | **Pre-Autoclave** | **Post-Autoclave** |
| **Assay (%)** | 100.7 | 101.0 | 98.9 | 100.3 |
| **Highest Unknown Impurity (%)** | 0.008 | 0.036 | 0.008 | 0.025 |
| **Total Impurities (%)** | 0.01 | 0.063 | 0.009 | 0.046 |

The use of polypropylene bags leads to a reduction in the highest impurity generated. The amount of highest impurity generated even at ambient conditions is lower than acetaminophen solution autoclaved in glass bottles and bubbled with nitrogen at levels less than 2 ppm.

### EXAMPLE 3

After autoclaving, the bags from Example 2, Method B were packaged with an overwrap containing an oxygen scavenger (D-100 FreshPax). The dissolved oxygen levels in the product were determined at various time points. Results are summarized below.

| **TABLE 4: DISSOLVED OXYGEN LEVELS IN ACETAMINOPHEN INJECTION, 10 MG/ML DRUG PRODUCT, PPM** | | | |
|---|---|---|---|
| | **INITIAL** | **96 HOURS** | **288 HOURS** |
| **Method A bag** | 8.89 | 1.44 | 0.87 |
| **Method B bag** | 8.95 | 1.02 | 0.68 |

The product after 288 hours is essentially identical to the product manufactured using Example 2, Method A.

### EXAMPLE 4

After autoclaving, the bags from Example 2, Method B were packaged with an overwrap containing an oxygen scavenger (D-100 FreshPax). The dissolved oxygen levels in the product were determined at various time points. Results are summarized below.

Compounding: Solutions of 0.1N hydrochloric acid (1000 mL) and 5N sodium hydroxide (5000 mL) were prepared for pH adjustment. A 600 L stainless steel compounding tank was rinsed with water for injection (WFI). The rinsing water was discarded and the tank was dried. The tank was then filled with 450L of WFI and maintained between 20°C and 25°C. To the WFI in the preparation tank, the required quantity of sodium chloride was added slowly under agitation and the agitation was continued until complete dissolution for a minimum of 10 minutes. The required quantity of citric acid anhydrous was added slowly to the preparation tank and agitated until complete dissolution for a minimum of 10 minutes. The pH of the solution was checked using a pH potentiometer. If needed, the pH of the solution was adjusted to be between 5.4 and 5.6 (target: 5.5) using either 0.1N hydrochloric acid or 5 N sodium hydroxide solution. The solution was agitated for a minimum of 5 minutes between each addition. The solution was mixed for another 10 minutes. Under slow agitation, the solution was heated to a temperature between 35°C and 45°C (target: 40°C), and mixing continued until dissolved oxygen levels were ≥ 3 ppm. Under agitation, the required amount of acetaminophen was added and agitated until completely dissolved, for a minimum of 60 minutes. The solution was then cooled to a temperature between 20°C and 25°C. The solution was brought up to final volume using WFI and agitated for a minimum of 10 minutes.

Filtration for Bioburden Reduction: Filtered compressed air was connected to the compounding tank and with a pressure between 1.5 - 3.0 bar the solution was filtered using a 0.22 um Millipore PVDF filter and filled into polypropylene bags.

Terminal Sterilization: The terminal sterilization parameters were as follows: sterilization temperature, 121.1°C; sterilization time, 20 minutes; and sterilization pressure, 0.5 bar.

The autoclaved bags were placed into an aluminum overpouch along with one scavenger packet (D-100 FreshPax) and immediately sealed.

The in-process assay, filling line evaluation and finished product data are summarized below.

| **TABLE 5: IN PROCESS AND FINAL PRODUCT DATA FOR EXHIBIT BATCH** | | | | | | | |
|---|---|---|---|---|---|---|---|
| | **SPEC** | **Compounding** | | **Filling** | | | **Terminal Sterilization** |
| | | *Top of Tank* | *Bottom of Tank* | *Beginning* | *Middle* | *End* | |
| **Appearance** | CCS | CCS | CCS | - | - | - | CCS |
| **pH, In-process** | 5.40-5.60 | 5.55 | 5.55 | 5.51 | 5.52 | 5.54 | - |
| **pH, Product** | 5.0-6.0 | - | - | - | - | - | 5.51 |
| **Avg fill volume (mL)** | NLT 100 mL | - | - | 103.6 | 103.2 | 103.3 | 102 |
| **Osmolality** | 250-320 | | | | | | 301 |
| **particulate matter, ≥ 10 µM** | NMT 600 | - | - | 267 | 280 | 267 | 813 |
| **particulate matter, ≥ 25 µM** | NMT 6000 | - | - | 0 | 20 | 73 | 20 |
| **Assay (%)** | 90.0-110.0 | 100.1 | 99.9 | 102.0 | 101.0 | 101.0 | 101.9 |
| **p-amino phenol, %** | NMT 0.05 | ND | ND | ND | ND | ND | ND |
| **highest unknown impurity, %** | NMT 0.10 | 0.01 | 0.01 | 0.01 | 0.02 | 0.01 | 0.05 |
| **total impurities, %** | NMT 0.3 | 0.01 | 0.02 | 0.01 | 0.03 | 0.01 | 0.12 |
| **Bioburden** | NMT 50 CFU/mL | 0 | 0 | - | - | - | - |
| **Sterility** | Sterile | - | - | - | - | - | Sterile |
| **Endotoxin** | NMT 0.35 EU/mg | <0.050 | <0.050 | - | - | - | <0.050 |
| *ND: not detected; NMT: not more than; NLT: not less than; CCS: clear colorless solution* | | | | | | | |

Dissolved oxygen was measured during the compounding process; results are summarized below.

| **TABLE 6: DISSOLVED OXYGEN LEVEL IN BULK DURING COMPOUNDING PROCESS** | |
|---|---|
| **Compounding Step** | **Dissolved Oxygen Level (ppm)** |
| Water for Injection addition to compounding tank | 1.74 |
| After dissolution of Sodium Chloride | 0.06 |
| After dissolution of citric acid | 8.82 |
| After dissolution of Acetaminophen | 8.54 |
| After addition of water for injection and final q.s. | 8.36 |

Impurities were monitored at the bottom of the tank over a 72-hour period; results are summarized below.

| **TABLE 7: BULK HOLD STUDY DATA** | | | | | |
|---|---|---|---|---|---|
| | **SPEC** | **INITIAL** | **24 Hours** | **48 Hours** | **72 Hours** |
| **Appearance** | CCS | CCS | CCS | CCS | CCS |
| **Bulk pH** | 5.40-5.60 | 5.55 | 5.53 | 5.52 | 5.55 |
| **Assay (%)** | 90.0-110.0 | 99.9 | 100.7 | 102.6 | 101.7 |
| **p-acetamino-phenol impurity,** % | NMT 0.05 | ND | ND | ND | ND |
| **highest unknown impurity, %** | NMT 0.10 | 0.01 | 0.01 | 0.02 | 0.05 |
| **total impurities, %** | NMT 0.3 | 0.02 | 0.04 | 0.04 | 0.13 |
| **Bioburden** | NMT 50 CFU/mL | 0 | 0 | 0 | 0 |
| **Bacterial Endotoxin** | NMT 0.35 EU/mg | <0.050 | <0.050 | <0.050 | <0.050 |
| *ND: not detected; NMT: not more than; NLT: not less than; CCS: clear colorless solution* | | | | | |

The bulk solution was stable at least up to 72 hours.

## Claims

1. A process of manufacturing a pharmaceutically acceptable product comprising an aqueous based isotonic solution of acetaminophen comprising about 2 mM to about 500 mM of a buffering agent and having a pH of about 5 to about 6, said acetaminophen solution being contained in a moist heat sterilizable container which is a flexible infusion bag made of a plastic material, said process comprising sterilizing said flexible infusion bag filled with the acetaminophen solution by moist heat sterilization performed at a minimum temperature of about 121 °C, for a time of about 10 minutes to about 30 minutes and an air overpressure between about 0.2 bar to about 1.2 bar, and wherein the water is not deoxygenated and a nitrogen blanket is not used during formulation, or the formulation is stored in ambient conditions in the polymer bag before autoclaving.

2. The process of claim 1 wherein said flexible infusion bag is made of a plastic material which is one of a cycloolefinic polymer, a polypropylene polymer, a polyvinyl chloride polymer, and any combination thereof.

3. The process of claim 1, wherein the fill volume of said moist heat sterilizable container is about 20 ml to about 1000 ml.

4. The process of claim 1, wherein said buffering agent is selected from an acetate buffer, a citrate buffer, a borate buffer, a phosphate buffer, a maleic buffer, a succinic buffer, a tartaric buffer, a phthalate buffer, a formate buffer, a tris buffer, and any combination thereof.

5. The process of claim 1 wherein said aqueous based isotonic solution of acetaminophen comprises the following ingredients:
- 10.00 mg/mL of acetaminophen;
- 1.93 mg/mL of citric acid;
- 6.40 mg/mL of sodium chloride;
- sodium hydroxide and hydrochloric acid to adjust pH to 5.5; and
- water for injection.

6. The process of claim 1, wherein said moist heat sterilizable container is enclosed within one or more overwraps.

7. The process of claim 6, wherein said one or more overwraps comprise one or more of oxygen scavengers and moisture scavengers.

8. The process of claim 6, wherein said one or more overwraps is configured to provide a barrier to ingress of one or more of oxygen, moisture, and light to said acetaminophen solution within said moist heat sterilizable container.

9. The process of claim 1, wherein said moist heat sterilization of said moist heat sterilizable container with said acetaminophen solution is performed by one of water cascade sterilization and steam sterilization.

## Patentansprüche

1. Verfahren zur Herstellung eines pharmazeutisch verträglichen Produktes, das eine wässrig-basierte isotonische Acetaminophen-Lösung umfasst, die etwa 2mM bis etwa 500 mM eines Puffers umfasst und einen pH von etwa 5 bis etwa 6 hat, wobei die Acetaminophen-Lösung in einem durch feuchte Hitze sterilisierbaren Behälter enthalten ist, welcher ein elastischer Infusionsbeutel ist, der aus einem Kunststoffmaterial hergestellt ist, wobei das Verfahren Sterilisieren des elastischen Infusionsbeutels, der mit der Acetaminophen-Lösung gefüllt ist, mittels Sterilisation durch feuchte Hitze, die bei einer Mindesttemperatur von etwa 121°C für eine Zeit von etwa 10 Minuten bis etwa 30 Minuten und bei einem Luftüberdruck zwischen etwa 0,2 bar und etwa 1,2 bar durchgeführt wird, umfasst, und wobei das Wasser nicht desoxygeniert wird und keine Stickstoffdecke während der Formulierung verwendet wird, oder die Formulierung bei Umgebungsbedingungen in dem Polymerbeutel vor Autoklavieren gelagert wird.

2. Verfahren nach Anspruch 1, wobei der elastische Infusionsbeutel aus einem Kunststoffmaterial hergestellt ist, das eins von einem cycloolefinischen Polymer, einem Polypropylenpolymer, einem Polyvinylchloridpolymer und einer Kombination davon ist.

3. Verfahren nach Anspruch 1, wobei das Füllvolumen des durch feuchte Hitze sterilisierbaren Behälters etwa 20 ml bis etwa 1000 ml ist.

4. Verfahren nach Anspruch 1, wobei der Puffer aus einem Acetatpuffer, einem Citratpuffer, einem Boratpuffer, einem Phosphatpuffer, einem Maleinsäurepuffer, einem Bersteinsäurepuffer, einem Weinsäurepuffer, einem Phthalatpuffer, einem Formiatpuffer, einem Trispuffer und einer Kombination davon ausgewählt wird.

5. Verfahren nach Anspruch 1, wobei die wässrig-basierte Aceaminophen-Lösung die folgenden Ingredientien umfasst:
10,00 mg/ml Acetaminophen;
1,93 mg/ml Citronensäure;
6,40 mg/ml Natriumchlorid;
Natriumhydroxid und Salzsäure, um den pH auf 5,5 einzustellen, und
Wasser zur Injektion.

6. Verfahren nach Anspruch 1, wobei der durch feuchte Hitze sterilisierbare Behälter in einer oder mehreren Umverpackung(en) eingeschlossen ist.

7. Verfahren nach Anspruch 6, wobei die eine Umverpackung oder die mehreren Umverpackungen einen oder mehrere von Sauerstofffängern und Feuchtigkeitsfängern umfasst (umfassen).

8. Verfahren nach Anspruch 6, wobei die eine oder mehreren Umverpackung(en) konfiguriert ist (sind), um eine Barriere gegen Eindringen von einem oder mehreren von Sauerstoff, Feuchtigkeit und Licht in die Acetaminophen-Lösung in dem durch feuchte Hitze sterilisierbaren Behälter bereitzustellen.

9. Verfahren nach Anspruch 1, wobei die Sterilisation durch feuchte Hitze des durch feuchte Hitze sterilisierbaren Behälters mit der Acetaminophen-Lösung durch eins von Wasserkaskaden-Sterilisation und Dampfsterilisation durchgeführt wird.

## Revendications

1. Procédé de fabrication d'un produit pharmaceutiquement acceptable comprenant une solution d'acétaminophène isotonique de base aqueuse comprenant environ 2 mM à environ 500 mM d'un agent tampon et présentant un pH d'environ 5 à environ 6, ladite solution d'acétaminophène étant contenue dans un récipient stérilisable par chaleur humide qui est une poche de perfusion souple composée d'un matériau plastique, ledit procédé comprenant la stérilisation de ladite poche de perfusion souple remplie de la solution d'acétaminophène par une stérilisation par chaleur humide effectuée à une température minimale d'environ 121 °C, pendant une durée d'environ 10 minutes à environ 30 minutes et une surpression d'air entre environ 0,2 bar et environ 1,2 bar, et dans lequel l'eau n'est pas désoxygénée et une atmosphère d'azote n'est pas utilisée pendant la formulation, ou la formulation est stockée dans des conditions ambiantes dans la poche polymère avant autoclavage.

2. Procédé selon la revendication 1 dans lequel ladite poche de perfusion souple est composée d'un matériau plastique qui est l'un parmi un polymère cyclooléfinique, un polymère de polypropylène, un polymère de polychlorure de vinyle, et toute combinaison de ceux-ci.

3. Procédé selon la revendication 1, dans lequel le volume de remplissage dudit récipient stérilisable par chaleur humide est d'environ 20 ml à environ 1000 ml.

4. Procédé selon la revendication 1, dans lequel ledit agent tampon est sélectionné parmi un tampon d'acétate, un tampon de citrate, un tampon de borate, un tampon de phosphate, un tampon maléique, un tampon succinique, un tampon tartrique, un tampon de phtalate, un tampon de formate, un tampon tris, et toute combinaison de ceux-ci.

5. Procédé selon la revendication 1 dans lequel ladite solution d'acétaminophène isotonique de base aqueuse comprend les ingrédients suivants :
- 10,00 mg/ml d'acétaminophène ;
- 1,93 mg/ml d'acide citrique ;
- 6,40 mg/ml de chlorure de sodium ;
- de l'hydroxyde de sodium et de l'acide chlorhydrique pour ajuster le pH à 5,5 ; et
- de l'eau pour injection.

6. Procédé selon la revendication 1 dans lequel ledit récipient stérilisable par chaleur humide est enfermé dans une ou plusieurs surenveloppes.

7. Procédé selon la revendication 6, dans lequel lesdites une ou plusieurs surenveloppes comprennent un ou plusieurs parmi des éliminateurs d'oxygène et des éliminateurs d'humidité.

8. Procédé selon la revendication 6, dans lequel lesdites une ou plusieurs surenveloppes sont configurées pour fournir une barrière à l'entrée d'un ou plusieurs éléments parmi de l'oxygène, de l'humidité et de la lumière dans ladite solution d'acétaminophène dans ledit récipient stérilisable par chaleur humide.

9. Procédé selon la revendication 1, dans lequel ladite stérilisation par chaleur humide dudit récipient stérilisable par chaleur humide avec ladite solution d'acétaminophène est effectuée par l'une parmi une stérilisation en cascade d'eau et une stérilisation à la vapeur.
